(19) Europäisches Patentamt
European Patent Office
Office européen des brevets

(11) Numéro de publication : **0 045 980**
**B1**

# (12) FASCICULE DE BREVET EUROPÉEN

(45) Date de publication du fascicule du brevet : **05.10.83**

(51) Int. Cl.³ : **C 07 D401/04**

(21) Numéro de dépôt : **81107653.8**

(22) Date de dépôt : **17.06.80**

(60) Numéro de publication de la demande initiale en application de l'article 76 CBE : **0021973**

## (54) Composés utiles notamment pour la préparation d'aminopipéridines ayant des propriétés anorexigènes.

(30) Priorité : **21.06.79 FR 7915974**

(43) Date de publication de la demande : **17.02.82 Bulletin 82/07**

(45) Mention de la délivrance du brevet : **05.10.83 Bulletin 83/40**

(84) Etats contractants désignés : **AT BE CH DE FR GB IT LI LU NL SE**

(56) Documents cités : **Néant**

(73) Titulaire : **SANOFI, société anonyme**
**40, Avenue George V**
**F-75008 Paris (FR)**

(72) Inventeur : **Nisato, Dino**
**Viale Golgi 80**
**Pavia (IT)**
Inventeur : **Carminati Paolo**
**Via Pacini 24**
**Milano (IT)**

(74) Mandataire : **Combe, André et al**
**CABINET BEAU DE LOMENIE 55 rue d'Amsterdam**
**F-75008 Paris (FR)**

**EP 0 045 980 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Composés utiles notamment pour la préparation d'aminopipéridines ayant des propriétés anorexigènes

Dans la demande de brevet européen 0 021 973, on a décrit des 4-amino-1-(2-pyridyl)pipéridines de formule générale

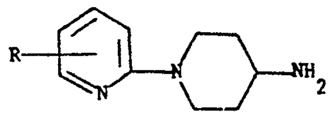

(I)

dans laquelle R représente de l'hydrogène, un halogène, un groupe méthyle, un groupe trifluorométhyle, un groupe alkoxy inférieur, un groupe trifluorométhoxy, un groupe 2,2,2-trifluoroéthoxy, un groupe alkylthio inférieur, un groupe trifluorométhylthio, un groupe phénoxy, un groupe phénoxy substitué par un halogène, un groupe trifluorométhyle, un alkyle inférieur, un alkoxy inférieur, un alkylthio inférieur ou un groupe cyano, un groupe phénylthio ou un groupe phénylthio substitué dans le noyau phényle par un atome d'halogène, un groupe trifluorométhyle, un alkyle inférieur, un alkoxy inférieur, un alkylthio inférieur ou un groupe cyano ; ainsi que leurs sels pharmaceutiquement acceptables.

Les termes « alkyle inférieur », « alkoxy inférieur » et « alkylthio inférieur », tels qu'utilisés ici, désignent des groupes contenant le résidu d'un hydrocarbure aliphatique saturé contenant 1 à 3 atomes de carbone, à savoir méthyle, éthyle, propyle et isopropyle.

Le terme « halogène » indique l'un des quatre halogènes communs, le fluor, le chlore et le brome étant particulièrement préférés.

Les sels pharmaceutiquement acceptables de ces 4-amino-1-(2-pyridyl)pipéridines comprennent les sels non toxiques dérivés d'acides minéraux ou organiques, salifiant une ou bien les deux fonctions basiques présentes dans la molécule des composés de formule I ci-dessus, tels que les chlorhydrates, les bromhydrates, les succinates, les tartrates, les citrates, les fumarates, les maléates, les pamoates, les napsylates, les mésylates, les tosylates.

Les nouvelles aminopipéridines de formule I possèdent une activité anorexigène remarquable et elles peuvent donc être utilisées pour le traitement de l'obésité.

On a trouvé que l'activité anorexigène des nouvelles aminopipéridines de formule I est sélective et ne comporte donc pas d'effets secondaires au niveau du système nerveux central. En particulier, les nouveaux composés de l'invention agissent sur le système sérotoninergique central sans effet sur le système dopaminergique et avec une action modérée sur le système noradrénergique.

Les nouvelles 4-amino-1-(2-pyridyl)pipéridines sont préparées par hydrolyse des 4-amino-1-(2-pyridyl)pipéridine N-substituées de formule

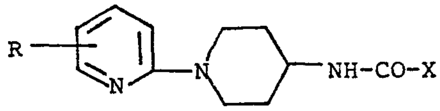

(V)

dans laquelle R et X r[illegible]ignifications données ci-dessus, dans des conditions acides ou basiques.

La présente in[illegible]erne en tant que produits nouveaux les composés de formule

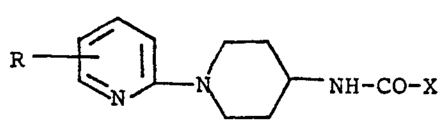

(V)

dans laquelle R représente de l'hydrogène, un halogène, un groupe méthyle, un groupe trifluorométhyle, un groupe alkoxy en $C_1$-$c_3$, un groupe trifluorométhoxy, un groupe 2,2,2-trifluoroéthoxy, un groupe alkylthio en $C_1$-$C_3$, un groupe trifluorométhylthio, un groupe phénoxy, un groupe phénoxy substitué dans le noyau phényle par un halogène, un groupe trifluorométhyle, un alkyle en $C_1$-$C_3$, un alkoxy en $C_1$-$C_3$, un alkylthio en $C_1$-$C_3$ ou un groupe cyano, un groupe phénylthio, ou un groupe phénylthio substitué dans le noyau phényle par un halogène, un groupe trifluorométhyle, un alkyle en $C_1$-$C_3$, un alkoxy en $C_1$-$C_3$, un alkylthio en $C_1$-$C_3$ ou un groupe cyano, et X représente un atome d'hydrogène ou un groupe alkyle en $C_1$-$C_4$.

La présente invention concerne également un procédé de préparation des produits de formule V, ce procédé étant caractérisé en ce que l'on fait réagir une 2-halopyridine de formule

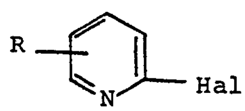

(III)

avec une aminopipéridine bloquée de formule

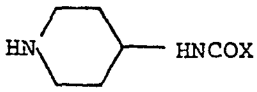 (IV)

dans laquelle X représente un atome d'hydrogène ou un groupe alkyle inférieur (ayant de 1 à 4 atomes de carbone), de façon à obtenir un produit selon l'invention.

La 4-acétamidopipéridine (formule IV, X = méthyle) représente la 4-aminopipéridine bloquée préférée.

La réaction de la 2-halopyridine de formule III avec la 4-aminopipéridine bloquée est conduite dans un solvant organique tel qu'un alcool, n-butanol, n-pentanol ou n-hexanol, de préférence diméthylformamide, diméthylsulfoxyde, sulfolane, acétonitrile, pyridine et similaires, à une température entre 50 et 200 °C, en présence d'un agent de condensation alcalin, tel qu'un hydroxyde, un carbonate ou un bicarbonate alcalin. Après 20 à 120 h, la réaction est complète et la 4-amino-1-(2-pyridyl)pipéridine N-substituée de formule V ainsi obtenue est isolée selon les techniques habituelles.

Pour accélérer la réaction, il peut être opportun d'opérer en présence d'un catalyseur, par exemple en présence de cuivre.

Les 4-amino-1-(2-pyridyl)pipéridines N-substituées de formule V sont des produits nouveaux.

Les produits de l'invention peuvent être utilisés notamment pour la préparation de produits de formule I grâce à une hydrolyse acide ou basique.

Cette hydrolyse acide est effectuée, par exemple, en chauffant la 4-amino-1-(2-pyridyl)pipéridine bloquée de formule V dans une solution aqueuse d'un acide minéral ou organique, acide chlorhydrique de préférence, et la 4-amino-1(2-pyridyl)-pipéridine de formule I est isolée selon des techniques conventionnelles.

L'hydrolyse en milieu basique est effectuée par traitement du composé de formule V avec une base minérale, hydroxyde de sodium de préférence. A la fin de l'hydrolyse, la 4-amino-1-(2-pyridyl) pipéridine est isolée et/ou transformée dans ses sels d'addition par traitement avec une solution aqueuse hydroalcoolique ou dans un solvant organique de l'acide salifiant.

Les 4-aminopipéridines bloquées de formule IV employées comme produits de départ peuvent être préparées selon des méthodes connues en littérature, par exemple par réduction catalytique des 1-benzyl-4-aminopipéridines bloquées correspondantes ou par réduction des 4-aminopyridines bloquées correspondantes.

La 4-acétamidopipéridine, produit de départ préférentiel, est décrite dans le brevet des Etats-Unis d'Amérique n° 3 124 586.

Les exemples non limitatifs suivants illustrent l'invention.

### Exemple 1

On chauffe au reflux sous agitation pendant 40 h une suspension de 175,5 g de 4-acétamidopipéridine, 269,7 g de carbonate de potassium et 182,5 g de 2,6-dichloropyridine dans 1 600 ml d'alcool n-pentylique. On refroidit le mélange réactionnel, on élimine le solvant sous pression réduite et on reprend le résidu dans 1 000 ml d'eau. On extrait le produit solide dans 2 000 ml de chlorure de méthylène, on lave la phase organique avec de l'eau, on la sèche sur du sulfate de sodium anhydre et on l'évapore à sec. On reprend le résidu dans 1 200 ml d'éther diisopropylique, on agite pendant 30 min, on filtre et on lave avec de l'éther diisopropylique. On obtient 246 g de 4-acétamido-1-(6-chloro-2-pyridyl)pipéridine, F. 153-155 °C.

### Exemple 2

Suivant le mode opératoire décrit à l'exemple 1, par réaction de la 4-acétamidopipéridine avec la 2,3-dichloropyridine, on obtient, avec un rendement de 70 %, la 4-acétamido-2-(3-chloropyridyl)pipéridine qui, après cristallisation dans de l'acétate d'éthyle, fond à 118-120 °C.

### Exemple 3

On chauffe sous agitation à 130 °C, pendant 50 h, un mélange de 0,088 mole de 4-acétamidopipéridine, 15 g de carbonate de potassium, 0,088 mole de 2-chloro-6-méthoxypyridine dans 100 ml de diméthylsulfoxyde, puis on refroidit, on verse le mélange dans de l'eau et on extrait la suspension ainsi obtenue avec de l'éther diéthylique. On extrait la phase aqueuse avec du chlorure de méthylène, on lave la phase organique avec de l'eau, on la sèche sur du sulfate de sodium anhydre et on l'évapore à sec. On obtient ainsi la 4-acétamido-1-(6-méthoxy-2-pyridyl)pipéridine qui, après cristallisation dans de l'éthanol à 95 %, fond à 125-127 °C. Rendement : 43 % de la théorie.

Exemple 4

On chauffe sous agitation, pendant 50 h, à 130 °C, un mélange de 12,5 g de 4-acétamidopipéridine, 15 g de carbonate de potassium, 10 g de 2-chloropyridine dans 100 ml de diméthylsulfoxyde, puis on le refroidit, on le verse dans l'eau et on extrait la suspension ainsi obtenue avec de l'éther diéthylique. On extrait la phase aqueuse avec du chlorure de méthylène, on lave la phase organique avec de l'eau, on la sèche sur du sulfate de sodium anhydre et on l'évapore à sec. On obtient ainsi la 4-acétamido-1-(2-pyridyl)-pipéridine qui, après cristallisation dans de l'isopropanol et recristallisation dans de l'acétate d'éthyle, fond à 165-168 °C. Rendement : 4,7 g (24,5 % de la théorie).

Exemple 5

On chauffe à 150 °C sous agitation, pendant 40 h, un mélange de 0,116 mole de 2-chloro-6-phénoxypyridine, 21,5 g de carbonate de potassium anhydre, 0,151 mole de 4-acétamidopyridine et 0,118 mole de cuivre en poudre dans 100 ml de sulfolane. On distille le sulfolane, on reprend le résidu dans de l'eau, on traite la suspension avec de l'eau et on l'extrait dans du chlorure de méthylène. On lave la phase organique avec de l'eau, on la sèche et on la concentre jusqu'à siccité. Par cristallisation du résidu dans de l'éther diisopropylique, on obtient la 4-acétamido-2-(6-phénoxy-2-pyridyl)pipéridine ; F. 104-106 °C. Rendement 30 % de la théorie.

Exemples 6 à 8

Suivant le mode opératoire décrit à l'exemple 5, par réaction de la 4-acétamidopipéridine avec la 2-chloro-4-méthylpyridine, la 2-chloro-6-(méthylthio)pyridine et la 2-chloro-6-(isopropylthio(pyridine (E. 130 °C/16 mmHg, préparée par réaction de la 2,6-dichloropyridine avec l'isopropylmercaptan, sel de Na), on obtient les 4-acétamido-1-(2-pyridyl)pipéridines correspondantes. Les caractéristiques physico-chimiques et les rendements des réactions sont résumés dans le tableau I suivant.

TABLEAU I

| Exemple | Produits de l'invention<br>Composé | Rendement<br>% | F., °C |
|---|---|---|---|
| 6 | 4-acétamido-1-(4-méthyl-2-pyridyl)pipéridine | 48 | 156-159 |
| 7 | 4-acétamido-1-(6-méthylthio-2-pyridyl)pipéridine | 39 | 128-130 |
| 8 | 4-acétamido-1-(6-isopropylthio-2-pyridyl)pipéridine | 64 | 115-117 |

Exemple 9

On chauffe à 150 °C sous agitation, pendant 50 h, un mélange de 9,5 g de 2-chloro-6-(phénylthio)pyridine (E. 112-116 °C/0,01 mmHg, préparée par réaction de la 2,6-dichloropyridine avec le sel sodique du thiophénol), de 7,8 g de carbonate de potassium et de 7,6 g de 4-acétamidopyridine dans 75 ml de sulfolane, puis on concentre le sulfolane à 1/5 de son volume, on verse le mélange dans 100 ml d'eau et on extrait 4 fois avec 150 ml d'éther diéthylique. On lave la phase organique avec de l'eau, on la sèche sur du sulfate de sodium anhydre, on la concentre jusqu'à siccité et on cristallise le résidu dans 40 ml d'acétate d'éthyle. On obtient ainsi 6,8 g de 4-acétamido-1-(6-phénylthio-2-pyridyl)pipéridine ; F. 110-112 °C.

Exemple 10

On chauffe à 150 °C, sous agitation, pendant 24 h 0,042 mole de 2-chloro-6-méthylpyridine, 0,056 mole de carbonate de potassium anhydre et 0,052 mole de 4-acétamidopyridine dans 75 ml de sulfolane, puis on concentre le sulfolane jusqu'à 1/5 de son volume, on verse le mélange dans 100 ml d'eau et on l'extrait 4 fois avec 150 ml d'éther diéthylique. On lave la phase organique avec de l'eau, on la sèche sur du sulfate de sodium anhydre, on la concentre jusqu'à siccité et on cristallise le résidu dans 40 ml d'acétate d'éthyle. On obtient ainsi la 4-acétamido-1-(6-méthyl-2-pyridyl)pipéridine ; F. 145-147 °C. Rendement : 28 %.

Exemple 11

On chauffe à 100 °C, sous agitation, pendant 50 h, un mélange de 19 g de 2-chloro-6-(2,2,2-trifluoroéthoxy)pyridine (E. 80 °C/16 mmHg, préparée par réaction de la 2,6-dichloropyridine avec 2,2,2-trifluoroéthanolate de sodium), 14,87 g de 4-formamidopipéridine (F. 116-118 °C, préparée par hydrogénation de la 4-formamido-1-benzylpipéridine en présence de palladium sur du charbon), 17,27 g de carbonate de potassium anhydre, 5,67 g de cuivre en poudre dans 110 ml de sulfolane, puis on distille le sulfolane, on reprend le résidu dans de l'eau et on extrait avec du chlorure de méthylène la suspension ainsi obtenue. On lave la phase organique avec de l'eau, on la sèche sur du sulfate de sodium anhydre et on l'évapore à sec. Le résidu, cristallisé d'abord dans un mélange eau/éthanol 84/16 en volume et après dans de l'éther diisopropylique, donne 9 g de 4-acétamido-1-[6-(2,2,2-trifluoro)éthoxy-2-pyridyl]pipéridine, F. 92-95 °C.

Exemple 12

On chauffe à 150 °C, sous agitation, pendant 20 h, un mélange de 0,042 mole de 2,6-dibromopyridine, 0,056 mole de carbonate de potassium anhydre et 0,052 mole de 4-acétamidopipéridine dans 75 ml de sulfolane, puis on concentre le sulfolane jusqu'à 1/5 de son volume, on verse le mélange réactionnel dans 100 ml d'eau et on extrait avec de l'éther diéthylique (4 × 150 ml). On lave la phase organique avec de l'eau, on la sèche sur du sulfate de sodium anhydre, on la concentre jusqu'à siccité et on cristallise le résidu dans 40 ml d'acétate d'éthyle. On obtient ainsi la 4-acétamido-1-(6-bromo-2-pyridyl)pipéridine ; F. 158-160 °C. Rendement : 56 % de la théorie.

**Revendications**

1. 4-amino-1-(2-pyridyl)pipéridines N-substituées de formule

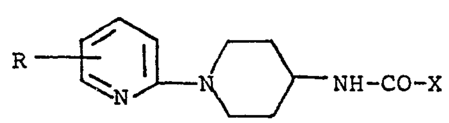

(V)

dans laquelle R représente de l'hydrogène, un halogène, un groupe méthyle, un groupe trifluorométhyle, un groupe alkoxy en $C_1$-$C_3$, un groupe trifluorométhoxy, un groupe 2,2,2-trifluoroéthoxy, un groupe alkylthio en $C_1$-$C_3$, un groupe trifluorométhylthio, un groupe phénoxy, un groupe phénoxy substitué dans le noyau phényle par un halogène, un groupe trifluorométhyle, un alkyle en $C_1$-$C_3$, un alkoxy en $C_1$-$C_3$, un alkylthio en $C_1$-$C_3$ ou un groupe cyano, un groupe phénylthio, ou un groupe phénylthio substitué dans le noyau phényle par un halogène, un groupe trifluorométhyle, un alkyle en $C_1$-$C_3$, un alkoxy en $C_1$-$C_3$, un alkylthio en $C_1$-$C_3$, ou un groupe cyano, et X représente un atome d'hydrogène ou un groupe alkyle en $C_1$-$C_4$.

2. 4-acétamido-1-(6-chloro-2-pyridyl)pipéridine.

3. Procédé de préparation d'un produit de formule V, caractérisé en ce que l'on fait réagir une 2-halopyridine de formule

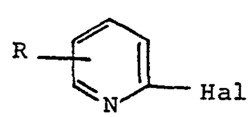

dans laquelle R a la signification ci-dessus et Hal représente un atome d'halogène avec une 4-aminopipéridine bloquée de formule

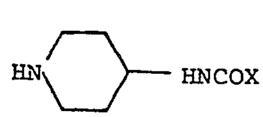

dans laquelle X représente un atome d'hydrogène ou un groupe alkyle en $C_1$-$C_4$.

4. Procédé selon la revendication 3, caractérisé en ce que comme 4-aminopipéridine bloquée, on utilise la 4-acétamidopipéridine.

5. Procédé selon l'une des revendications 3 et 4, caractérisé en ce que la réaction est effectuée en présence d'un catalyseur, par exemple le cuivre.

**Claims**

1. N-substituted 4-amino-1-(2-pyridyl)piperidines of formula

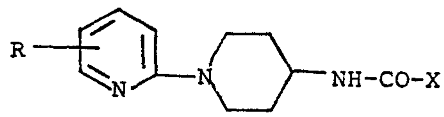

in which R represents hydrogen ; a halogen, a methyl group, a trifluoromethyl group ; a $C_1$-$C_3$ alkoxy group ; a trifluoromethoxy group ; a 2,2,2-trifluoroethoxy group ; a $C_1$-$C_3$ alkylthio group ; a trifluoromethylthio group ; a phenoxy group ; a phenoxy group substituted in the phenyl ring by a halogen, a trifluormethyl group, a $C_1$-$C_3$ alkyl, a $C_1$-$C_3$ alkoxy, a $C_1$-$C_3$ alkylthio or a cyano group ; a phenylthio group ; or a phenylthio group substituted in the phenyl ring by a halogen, a trifluoromethyl group, a $C_1$-$C_3$ alkyl, a $C_1$-$C_3$ alkoxy, a $C_1$-$C_3$ alkylthio or a cyano group ; and X represents an atom of hydrogen or a $C_1$-$C_4$ alkyl group.

2. 4-acetamido-1-(6-chloro-2-pyridyl)piperidine.

3. A process for the preparation of a product of formula V, characterized in that a 2-halopyridine of formula

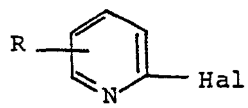

in which R has the above significance and Hal represents an atom of halogen is reacted with a blocked 4-aminopiperidine of formula

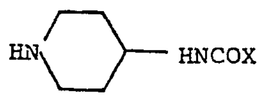

in which X represents an atom of hydrogen or a $C_1$-$C_4$ alkyl group.

4. A process according to claim 3, characterized in that 4-acetamidopiperidine is used as blocked 4-aminopiperidine.

5. A process according to any one of claims 3 and 4, characterized in that the reaction is carried out in the presence of a catalyst, such as copper.

**Ansprüche**

1. N-substituierte 4-Amino-1-(2-pyridyl)-piperidine der Formel

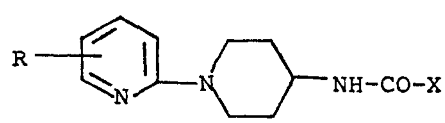

worin R für Wasserstoff, Halogen, Methyl, Trifluormethyl, $C_1$-$C_3$-Alkoxy, Trifluormethoxy, 2,2,2-Trifluoräthoxy, $C_1$-$C_3$-Alkylthio, Trifluormethylthio, Phenoxy, eine im Phenylkern durch Halogen, Trifluormethyl, $C_1$-$C_3$-Alkyl, $C_1$-$C_3$-Alkoxy, $C_1$-$C_3$-Alkylthio oder Cyano substituierte Phenoxygruppe, Phenylthio oder eine im Phenylkern durch Halogen, Trifluormethyl, $C_1$-$C_3$-Alkyl, $C_1$-$C_3$-Alkoxy, $C_1$-$C_3$-Alkylthio oder Cyano substituierte Phenylthiogruppe steht und X Wasserstoff oder $C_1$-$C_4$-Alkyl darstellt.

2. 4-Acetamido-1-(6-chlor-2-pyridyl)-piperidin.

3. Verfahren zur Herstellung eines Produktes der Formel V, dadurch gekennzeichnet, daß 2-Halogenpyridin der Formel

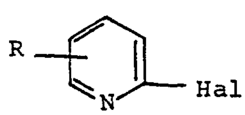

worin R obige Bedeutung hat und Hal ein Halogenatom darstellt, mit einem geschützten 4-Aminopiperidin der Formel

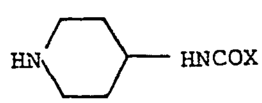

worin X Wasserstoff oder $C_1$-$C_4$-Alkyl darstellt, umgesetzt wird.

4. Verfahren nach Anspruch 3, dadurch gekennzeichnet, daß als geschütztes 4-Aminopiperidin 4-Acetamidopiperidin verwendet wird.

5. Verfahren nach einem der Ansprüche 3 und 4, dadurch gekennzeichnet, daß die Reaktion in Gegenwart eines Katalysators, beispielsweise Kupfer, durchgeführt wird.